# EUROPEAN PATENT APPLICATION

(11) **EP 4 552 563 A1**
(43) Date of publication of application: **14.05.2025**
(21) Application number: 24210881.9
(22) Date of filing: 05.11.2024
(51) Int. Cl.: A61B 5/103, A43B 3/34, A43B 17/00, A61B 5/11, A61B 5/00, G16H 40/67

(54) **SMART SENSING INSOLE**

(30) Priority: 10.11.2023 TW 112143543; 29.11.2023 US 202318522896
(71) Applicant: Decentralized Biotechnology Intelligence Co., Ltd., Taipei City, Da'an Dist. 106021 (TW)
(72) Inventor: Chou, Yao-Sheng, 106021 Taipei (TW); SU, Wei-Sheng, 106021 Taipei (TW)
(74) Representative: Zaboliene, Reda

(57) **Abstract**

The present invention discloses a smart sensing insole including a pressure sensing layer (for instance a pressure plate) which includes sensing sensors. The sensors are used as sensing points to measure the pressure and the distribution when the foot pressure changes. Each sensor is coupled to the sensing module. The area occupied by the sensing points is between 3-50% or 3-70% of the entire insole area. The sensing point position is configurated at pressure peak position, pressure center area, foot arch position.

## Description

### TECHNICAL FIELD

The present invention relates to an insole, more specifically, a smart sensing insole.

### BACKGROUND

According to medical literature and research, feet are most closely related to physical health. However, the feet bear the weight of the body. Plantar pressure reveals an important indicator of gait, and the pressure distribution has significant references in the fields of biomechanics, rehabilitation, sport training, shoemaking and other fields. The traditional plantar pressure testing benches have space limitations. For testing plantar pressure, the conventional sensors are in contact with the feet, and they cause uncomfortable to the user due to the sensors contact with the feet all the time. It is not conducive for long-term wearing and testing, and the conventional method cannot provide sufficient health information.

Prior art TW201729704A includes a pressure sensor, a temperature sensor, and a humidity sensor. The pressure sensor, temperature sensor and humidity sensor are formed on the surface of the insole rather than in the interlayer of the insole. As aforementioned, it is easy to cause sensor wear and is discomfort to the user. The length, width, and thickness of each sensor are 1mm × 3mm × 0.02mm. Obviously, each sensor is used as an individual device, therefore, it does not have mass production efficiency, and the sensors do not locate on key areas, therefore, the configuration is not arranged based on cost-effectiveness. This previous sensor is configurated randomly, it is unable to capture data at key locations, resulting in distortion.

With the rapid development of cloud computing, wireless communication and artificial intelligence, health systems integrating sensors, wireless device and intelligent computing have become the trends of research and development. Therefore, in view of this, what is required is to collect health information by these technologies. The present invention proposes a smart sensing insole to facilitate the assessment of plantar pressure.

### SUMMARY OF THE INVENTION

Based on above, one aspect of the present invention is to provide a sensing insole to completely measure foot information. The further purpose of the present invention is to provide a smart sensing insole to improve sensing efficiency. The present invention includes a pressure sensing layer. When the foot pressure changes, the sensors are employed to sense the pressure change and the pressure distribution. The area of sensing points occupies about 3-50% or 3-70% of the total insole bottom surface area. The sensing point locations include: pressure peak position, pressure center area or arch shape position, where the first position range (first priority) includes: the big toe area, the first toe joint area, the fifth toe joint area, the heel area; the second position range (secondary priority) includes: the middle toe joint area, the lateral longitudinal arch area near the heel, the middle area of the transverse arch, and the lateral longitudinal arch area close to the transverse arch. The third position range (third priority) includes: the medial longitudinal arch close to the transverse arch area, and the medial longitudinal arch close to the heel area.

According to another aspect of the present invention, the present invention includes a sensing module coupled to the pressure sensing layer to receive sensing data; the foot sensing module is configured in the arch of the smart sensing insole. The present invention may also include an inertial sensor, an infrared sensor, and a GPS, which are arranged in the arch of the smart sensing insole.

In another embodiment, the present invention includes a wireless transmission/reception module, coupled to the sensing module, and wirelessly coupled to an external mobile device. The foot information received and processed by the sensing module is displayed by the external mobile device. The foot information includes one or any combination of the following: foot pressure distribution, weight ratio of left and right feet, gait, cadence, foot pressure center. Foot information is uploaded to the big data database through the mobile devices, and the big data database uses blockchain as the communication structure.

In one embodiment, the sensing module is used to collect foot information and display it in real time through the mobile device to obtain personal foot pressure information and establish a relationship between the exercise and the foot pressure. The sensing module is connected to one or any combination of a pressure sensing device, an inertial sensor, an infrared sensor, an accelerometer, a gyroscope, and a GPS. All foot information is processed to obtain foot pressure distribution and foot blood circulation status data.

In another aspect of the present invention, the present invention can achieve accurate pressure and exercise measurement, no matter what kind of the exercise is, through accurate sensing data, it is conducive to exercise analysis. The present invention provides exercise sensing and management, and provides details of each process. Based on the above, the present invention proposes to solve the deficiencies of the existing technology. According to one of the viewpoints of the present invention, the present invention can be employed to collect foot information, and stores in a cloud big data database by the mobile devices.

### BRIEF DESCRIPTION OF THE DRAWINGS

Figure 1 shows the structure of the present invention.
Figure 2A shows the pressure sensing layer of the present invention.
Figure 2B shows the pressure sensing layer of the present invention.
Figure 3 shows smart sensing insole and smart phone diaphragm of the present invention.
Figure 4 shows the cloud system with the smart phone according to the present invention.

### DETAILED DESCRIPTION

Some preferred embodiments of the present invention will now be described in greater detail. However, it should be recognized that the preferred embodiments of the present invention are provided for illustration rather than limiting the present invention. In addition, the present invention can be practiced in a wide range of other embodiments besides those explicitly described, and the scope of the present invention is not expressly limited except as specified in the accompanying claims.

The present invention integrates artificial intelligence (AI) and dynamic sensing technology, the integrated design allows users to feel comfortable while recording and analyzing feet status, and the present invention provides users with the most complete health management information. The present invention may provide real-time feedback through APP, it not only contributes to comprehensive solutions of health management, but also analyzes various data through the exercise, thereby reducing the health risks. It is an indispensable tool for implementing exercise and health management. In one embodiment, the plantar pressure sensing of the present invention includes a pressure sensing plate. The pressure value is obtained during the measurement process, and the plantar pressure parameters and pressure distribution plots are obtained based on subsequent processing.

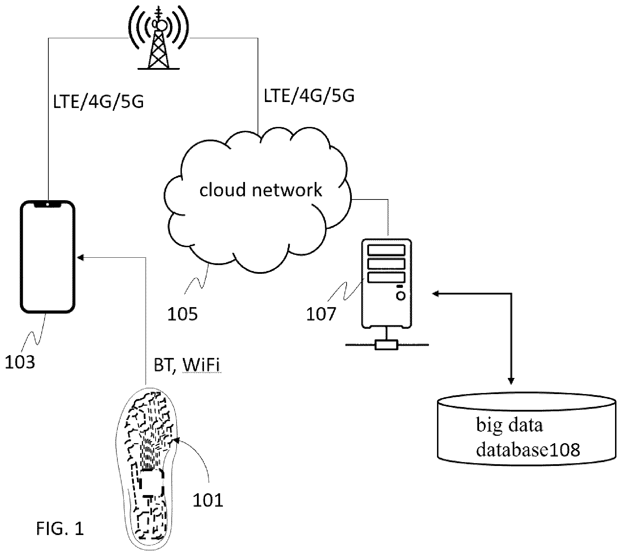

Figure 1 shows a schematic diagram of the present invention which includes a cloud server 107 electrically connected to a large data database 108. The present invention employs the smart sensing insole 101 to collect foot information and monitor the user's foot pressure, blood oxygen (described in detail later), etc. The smart sensing insole 101 can communicate with mobile devices (external computing electronics such as smart phones, tablets, etc.) 103. The present invention includes an application program installed in the mobile device 103, and the application program receives and sends data instructions between the smart sensing insole 101, the mobile device 103 and the cloud server 105. The above-mentioned applications operate based on Android, Windows or iOS operating system platforms, and upload the collected relevant data/signals to the cloud server 107 for storing and generating foot information through data analysis and computing, thereby providing health advices accordingly.

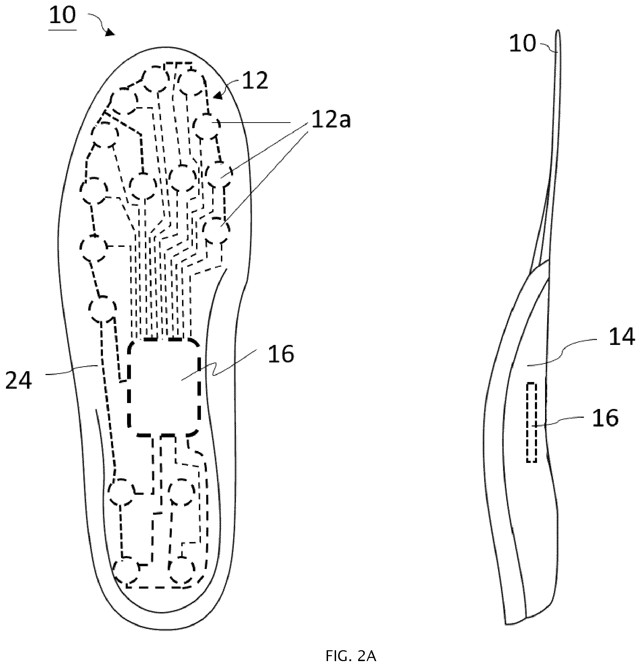

FIG. 2A shows a top view of the insole 10. The pressure sensing layer 12 is integrated within an insole 10 according to an embodiment of the present invention. The pressure sensing layer 12 is embedded inside the insole. The pressure sensing layer 12 includes a plurality of pressure sensors 12a, each individual pressure sensor 12a acts as a sensing point to sense the pressure, pressure change when the foot pressure changes. The pressure sensor 12a is electrically connected to the sensing module 16 through a wire 24. The shape of the pressure sensor 12a includes rectangular, triangle, circle, pentagon, hexagon or any suitable shape. In a preferred embodiment, the pressure sensor 12a is a resistance-type pressure sensor, when the sensor 12a is deformed by pressure, resulting in a resistant change, and the magnitude of the pressure can be estimated by measuring the change in resistance. The pressure sensing layer 12 is integrated within the of the insole 10. The insole 10 may include a thermoplastic polyester elastomer (TPEE) layer, and may also include an arch support (cushion) 14 integrated therein. A sensing module 16 is embedded in any location of the insole 10, in one embodiment, the sensing module 16 is formed in arch support 14 of the insole to collect, process and transmit the electronic signals of the pressure sensors 12a, thereby avoiding or minimizing the contact and stimulation to the wearer's foot. Preferably, the area of the sensing points is 3-50% or 3-70% of total insole bottom surface area.

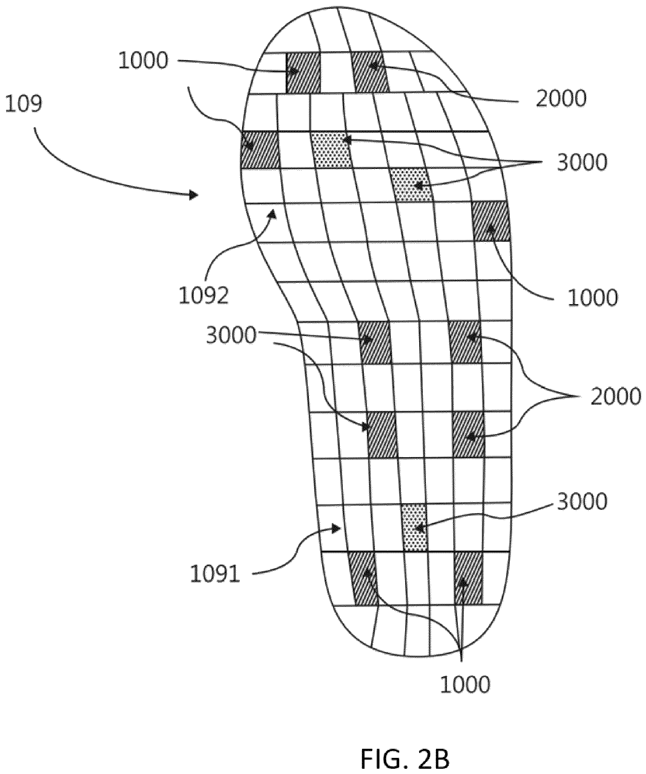

In one embodiment, the smart sensing insole 101 of the present invention includes a pressure sensing layer 109 (as shown in Figure 2) for detecting plantar pressure, left and right foot pressure distribution, gait cadence. In one embodiment, a pressure plate may be used. The present invention has a pressure sensing layer 109 embedded in the insole. The pressure sensing layer 109 includes a sensing array configuration composed of longitudinal conductive lines 1091 and transverse conductive lines 1092. The intersection points of longitudinal conductive lines 1091 and transverse conductive lines 1092 serve as individual pressure sensing points (sensor), when the foot pressure changes, it determines the pressure value and pressure distribution. The pressure sensing layer 109 is electrically connected to the sensing module through wires. It should be note that the longitudinal conductive lines 1091 includes vertical conductive lines and the first inclined conductive lines, the first inclination angles to the vertical direction are between 1-30 degrees, and the transverse conductive lines 1092 includes a horizontal conductive lines and second inclined conductive lines, and the second inclination angles are between 1 and 30 degrees to the horizontal direction. The aforementioned longitudinal conductive lines 1091 and transverse conductive lines 1092 divide the insole area into at least 10-120 sections, depending on the laying sensing density. In one embodiment, by taking into account cost-effectiveness and better sensing density, 20-100 sections or 30-80 sections are formed. The configuration of the first and the second inclined conductive lines are provided to fit the sole shape. The longitudinal inclined conductive lines or the transverse inclined conductive lines may include straight lines or curve lines. According to a preferred embodiment, the area of the sensing point occupies 3-50% of the total insole bottom surface area, in another embodiment, the area is between 10-40%. This value is obtained through multiple experiments based on the experimental group and the control group. It verifies that the area range will not only cause discomfort to the user, but also not reduce the sensing performance. The present invention may collect the pressure distribution conditions, determine the pressure center position, and the standing center deviation. Further, the present invention may determine abnormal pressure distribution for correcting walking posture.

More sensors are not conducive to obtain preferred data, the sensors should be deployed where there is benefit. After research and testing, the pressure sensing position configuration can be divided into at least three position ranges. The main areas are the pressure peak positions, the pressure center area, and the locations where differences in arch shape are reflected. The first position range 1000 is the first priority deployment range which includes big toe area, first toe joint area, fifth toe joint area, and heel area. The second position range 2000 is the second priority deployment range including the middle toe joint area, the lateral longitudinal arch area near the heel, the middle area of the transverse arch, and the lateral longitudinal arch area close to the transverse arch. The third position range 3000 is the third priority deployment range including the area where the medial longitudinal arch is close to the transverse arch, and the medial longitudinal arch area near the heel area. According to the cost and benefit, the configuration and quantity can be determined according to above order. If there are more demands, they can be deployed in other areas outside the above three locations.

FIG. 2 is a schematic diagram for showing the longitudinal conductive lines 1091 and the transverse conductive lines 1092, it does not show that lines cross the ranges 1000, 2000, and 3000. However, in fact, the lines may cross above ranges.

The longitudinal conductive lines 1091 and the transverse conductive lines 1092 form an array configuration, and the intersections of the two lines 1091, 1092 forms pressure sensing points. In one embodiment, the pressure sensing layer 109 includes a resistive pressure sensing element. The resistive pressure sensing line is composed of a conductive polymer, and the conductive polymer changes resistance as the pressure changes. Applying force brings the conductive particles into contact, which increases the current through the sensing wire and the pressure value is calculated. Another embodiment uses capacitive pressure sensing which employs a diaphragm to separate vertical wires and horizontal wires. When the diaphragm is deformed by pressure, the gap between the diaphragm and the two wires changes, further causing changes in capacitance, and thereby fetching the pressure through the change of capacitance.

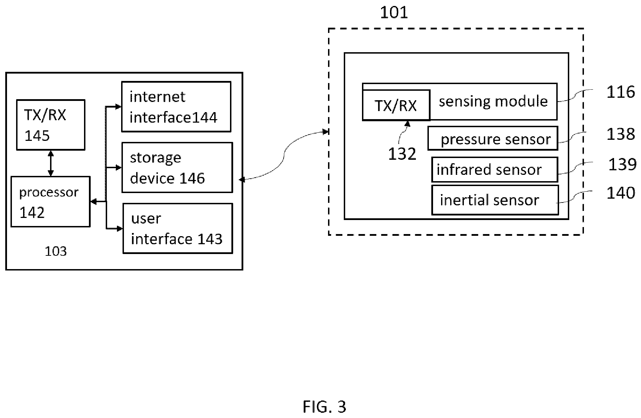

In one embodiment, as shown in Figure 3, the smart sensing insole 101 has a built-in inertial sensor 140. The inertial sensor 140 includes a three-axis accelerometer and a three-axis gyroscope to sense the static and dynamic physical values. The inertial sensor 140 can be disposed in the arch area of the insole, or in the interval formed by the interweaving of the longitudinal conductive lines 1091 and the transverse conductive lines 1092.

In another embodiment, the smart sensing insole 101 is equipped with an infrared sensor 139, which has red light/infrared light sources for blood oxygen and blood pressure detections. The blood pressure detection employs optical sensing of subcutaneous blood flow, and then blood pressure data is derived using well-known algorithms. The principle of blood oxygen transmission detection is that when the blood is sent to the periphery, there will be a slight volume change with the heart rate. It uses two light sources, red light and infrared, to pass through the tissue and receive the light by the sensors. The difference in light intensity caused by slight volume changes is converted into a signal and the blood oxygen concentration is calculated.

As shown in Figure 3, the smart sensing insole 101 can be the left foot or the right foot. These two are symmetrical structures. The illustration only shows one of them. It should be noted that it can be applied to both feet. The smart sensing insoles 101 of both feet are electrically connected to mobile devices 103, such as smart phones or tablets. Data can be received and sent by the wireless transmission/reception module 132. The wireless transmission/reception module 132 is compatible with wireless telecommunications standards, such as WiFi, Bluetooth, RFID, NFC, 5G or any other future wireless communication standards. Wireless transmission Module 132 is connected to the antenna to transmit and receive data.

The smart sensing insole 101 communicates with the external mobile device 103. The smart sensing insole 101 includes a foot sensing module 116 built into the arch of the insole to receive and analyze foot pressure distribution and foot blood circulation data, and transmits the data through the foot sensing module 116. The wireless transmission/reception (TX/RX) module 132 inside transmits the above data to a remote computing device or server.

The foot sensing module 116 executes software applications, and it includes a microprocessor and a storage unit. The microprocessor may be a microcontroller, a digital signal processor (DSP), an application specific integrated circuit (ASIC), a programmable logic circuit, or other digital data processing device that executes instructions to perform processing operations in accordance with the present invention. The microprocessor executes various application programs stored in the storage unit, including executing firmware algorithms. Storage units may include read only memory (ROM), random access memory (RAM), electrically erasable programmable ROM (EEPROM), flash memory, or any memory commonly used in computers.

FIG. 3 shows data transmission/reception of foot sensing module 116 signals through a wireless transmission/reception (TX/RX) module 132. In one embodiment, the wireless transmission/reception module 132 may be a wireless data transmission/reception device with Bluetooth, WiFi or similar functions. In other words, the wireless transmission/reception module 132 is compatible with wireless telecommunications standards, such as WiFi, Bluetooth, RFID, NFC, 5G or any other future wireless communication standards. The foot sensing module 116 can be electrically connected to the pressure sensor 138 in the pressure sensing layer 109 through the connection terminal, or connected to the infrared sensor 139 or the inertial sensor 140. The foot sensing module 116 also includes a processing system (eg, one or more microprocessors), memory, and the like.

The left or right smart sensing insole 101 includes additional sensors, such as accelerometers, gyroscopes, GPS, etc., and a power supply device to provide power to each component. It should be understood that the foot sensing module 116 can provide a computing programs/algorithms to collect, and analyze data (for example, foot pressure distribution data, pressure data interacting with the ground, or foot blood circulation status, etc.), and these programs/algorithms can be stored and/or executed.

The mobile device 103 includes a processor 142, a user interface 143, an internet interface 144 and a storage device 146, which are respectively connected to the processor 142. The user interface 143 includes one or more input devices (eg, touch screen, voice input device, etc.), one or more audio output devices (eg, speakers, etc.), and/or one or more visual output devices. The internet interface 144 includes one or more networking devices (eg, wireless local area network (WLAN) devices, wired LAN devices, wireless wide area network (WWAN) devices, etc.); storage device 146 includes flash memory devices. The wireless transmission/reception (TX/RX) module 145 performs data transmission and reception with the wireless transmission/reception (TX/RX) module 132.

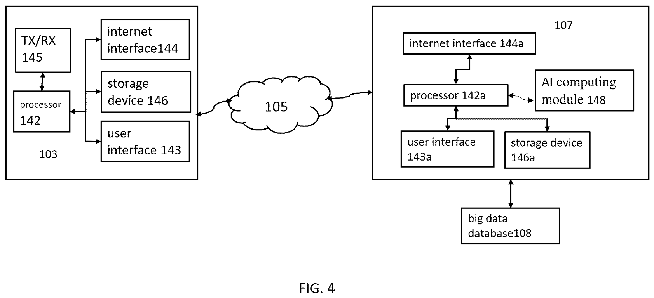

In one embodiment, the big data database 108 is connected to the cloud server 107. Referring to Figures 1 and 4, the big data database 108 is electrically connected to the AI computing module 148. In one embodiment, the AI computing module 148 installed in the cloud server 107 to analyze the data collected by the big data database 108. The AI algorithm includes a series of steps: pre-filtering and normalizing the input signal, extracting time domain and frequency domain features, and using a Convolutional Neural Networks (CNN) model to output classification results. Similarly, the cloud server 107 includes a user interface 143a, an internet interface 144a, a storage device 146a, and an individual connection processor 142a. In one embodiment, no matter what kind of exercise it is, accurate sensing data of the insole can be used to facilitate appropriate health management. Through the user's weight, speed, pressure and other data, AI analysis is performed to analyze the movement. The above is functioning that previous insole and sports watches cannot achieve.

In another perspective, the mobile device 103 combines an algorithm system to process data from sensors in the shoe, and analyzes pressure distribution, gait, cadence, center of pressure (COP), etc. The foot pressure distribution plays a key role in human movement. The foot shape and walking (running) posture affect human body posture and bone changes, as well as athletes' performance and limits. The insole with integrated sensors can obtain the parameter data of the foot pressure distribution of many users with respect to time and space by placing the insole in the shoe, and upload it to an external computer through wireless transmission. Devices, such as smartphones, personal computers, computer servers, etc., calculate and analyze and store them in cloud systems as relevant big data databases. Generally, traditional technology lacks visual/data-based learning standards to allow users to clearly realize every detail of the movement status. The present invention may provide detailed tracks during movement, and help users to realize the distribution of foot pressure, and thereby adjusting their walking posture.

In addition, the smart sensing insole disclosed by the present invention also integrates the infrared sensor 139 to simultaneously provide the user's blood circulation status information. Breaking through the previous limitation that only medical institutions or sports research institutions could obtain such analysis data, more users can obtain exclusive personal foot information by the present invention. In one embodiment, the above data is transmitted wirelessly and can be displayed in real time with the application APP, allowing above data to be visualized. The analytical data stored in the big data database 108 of the present invention can not only provide consumers with their own health management, but also provide data to other industries, or cross-industry, such as hospitals and shoemaking industry, for reference. In addition, the big data database 108 uses blockchain as the communication structure, the data cannot be changed, and the transmission is encrypted.

The present invention has a wireless charging induction coil, which is arranged on one side of the smart induction insole 101 to facilitate wireless charging and provide the power required by the smart induction insole, and the smart induction insole 101 is equipped with a rechargeable battery and a power supply module. In another embodiment, the wireless transmission/reception (TX/RX) module 132 can be replaced or coexisted by a USB (Universal Serial Bus) port for data transmission and wired charging.

As will be understood by persons skilled in the art, the foregoing preferred embodiment of the present invention illustrates the present invention rather than limiting the present invention. Having described the invention in connection with a preferred embodiment, modifications will be suggested to those skilled in the art. Thus, the invention is not to be limited to this embodiment, but rather the invention is intended to cover various modifications and similar arrangements included within the spirit and scope of the appended claims, the scope of which should be accorded the broadest interpretation, thereby encompassing all such modifications and similar structures. While the preferred embodiment of the invention has been illustrated and described, it will be appreciated that various changes can be made without departing from the spirit and scope of the invention.

## Claims

1. A smart sensing insole (101) comprising:
a sensing module (16) in said sensing insole (101);
a pressure sensing layer (109) including sensors (12a) to act as sensing points to sense foot pressure changes, wherein said sensors (12a) are coupled to said sensing module (16), area of said sensing points being 3-50% or 3-70% of total insole bottom surface area;
a wireless charging coil configured in said insole and coupled to said sensing module (116);
an inertial sensor (140) configured in said insole (101) and coupled to said sensing module (116); and
a wireless transmission/reception module (132) configured in said insole (101).

2. The insole of claim 1, wherein said inertial sensor (140) include an accelerometer, gyroscope or the combination thereof.

3. The insole of claim 1, wherein said insole (101) includes an infrared light sensor (139), a GPS or the combination thereof.

4. The insole of claim 1, wherein said wireless transmission/reception module (132) is compatible with WiFi, Bluetooth, RFID, NFC, 5G.

5. The insole of claim 1, wherein a foot information is displayed by an external device (103) coupled with said wireless transmission/reception module (132), wherein said foot information includes one or any combination of the following: foot pressure distribution, gait cadence, and pressure center.

6. The insole of claim 5, wherein said external device is coupled a cloud server (107) having an AI computing module (148).

7. The insole of claim 6, wherein said AI computing module (148) performs steps: pre-filtering and normalizing input signals, extracting time domain and frequency domain features.

8. The insole of claim 7, further using a Convolutional Neural Networks (CNN) model to output classification results.

9. The insole of claim 5, wherein said foot information is uploaded to a big data database (108) through said external device (103).

10. The insole of claim 9, wherein said big data database (108) employs blockchain as the communication structure to prevent said data from being changed.

11. The insole of claim 1, wherein said sensing points includes a pressure peak position, a pressure center area, and an arch position.

12. The insole of claim 11, wherein a first position range (1000) of said sensing points includes a big toe area, a first toe joint area, a fifth toe joint area, and a heel area.

13. The insole of claim 12, wherein a second position range (2000) of said sensing points includes a middle toe joint area, a middle area of a transverse arch, and a lateral longitudinal arch area near said transverse arch,

14. The insole of claim 13, wherein a third position range (3000) of said sensing points includes a medial longitudinal arch area near said transverse arch, said medial longitudinal arch area near said heel.

15. The insole of claim 1, wherein said sensing module (116) is configured in an arch portion of said insole (101).
